# EUROPEAN PATENT SPECIFICATION

(11) **EP 1 896 420 B1**
(45) Date of publication and mention of the grant of the patent: **18.01.2017**
(21) Application number: 06763731.4
(22) Date of filing: 14.06.2006
(51) Int. Cl.: C07D 217/10

(54) **PROCESS OF PRODUCING BLEACH BOOSTERS**
VERFAHREN ZUR HERSTELLUNG VON BLEICHAKTIVATOREN
PROCEDE DE PRODUCTION DE RENFORÇATEURS DE BLANCHIMENT

(30) Priority: 17.06.2005 EP 05013132; 17.06.2005 EP 05013136; 17.06.2005 EP 05013129
(43) Date of publication of application: 12.03.2008
(73) Proprietor: BASF SE, 67056 Ludwigshafen am Rhein (DE)
(72) Inventor: SCHEIN, Karin, 67065 Ludwigshafen (DE); KOCHNER, Arno, 67165 Waldsee (DE); VÖLKEL, Ludwig, 67117 Limburgerhof (DE); BITTNER, Christian, 64625 Bensheim (DE); MÜNSTER, Ingo, 67459 Böhl-Iggelheim (DE); DIETSCHE, Frank, 69198 Schriesheim (DE); SCHROF, Wolfgang, 67271 Neuleiningen (DE); ERNST, Hansgeorg, 67346 Speyer (DE); BECK, Karl, 76684 Östringen (DE); MISSKE, Andrea, 67346 Speyer (DE); SCHOLTISSEK, Martin, 67157 Wachenheim (DE)
(74) Representative: BASF IP Association
(86) International application number: PCT/EP2006/063237
(87) International publication number: WO 2006/134143

(56) References cited:
- WO-A-95/13353
- WO-A-98/16614
- WO-A-03/104199
- WO-A-2005/047264
- WO-A-2005/073360
- DE-A1- 19 507 522
- US-A1- 2006 089 284
- DATABASE CA [Online] CHEMICAL ABSTRACTS SERVICE, COLUMBUS, OHIO, US; GARKUSHA-BOZHKO, V. S. ET AL: "Reaction of pyridines with epichlorohydrin" XP002397773 retrieved from STN Database accession no. 1990:613851 & KHIMIYA GETEROTSIKLICHESKIKH SOEDINENII , (4), 507-10 CODEN: KGSSAQ; ISSN: 0453-8234, 1990,

## Description

This invention relates to a process of producing compounds, which are useful as bleach boosters, as well as to the compounds, which are obtainable using said process, and to their use.

Oxygen bleaching agents, for example hydrogen peroxide, are typically used to facilitate the removal of stains and soils from clothing and various surfaces. Unfortunately such agents are extremely temperature rate dependent. As a result, when such agents are employed in colder solutions, the bleaching action of such solutions is markedly decreased.

In an effort to resolve the aforementioned performance problem, the industry developed a class of materials known as "bleach activators" or "bleach boosters", which are also called "organic catalysts". However, as such materials rapidly lose their effectiveness at solution temperatures of less than 40 °C, new organic catalysts such as 3,4-dihydro-2-[2-(sulfooxy)decyl]isoquinolimium, inner salt were developed. In general, while such current art catalysts are effective in lower temperature water conditions, they can inactivate certain enzymes. As most laundry and cleaning compositions are formulated with enzymes, formulating cleaning products with such catalysts can be problematic.

Accordingly, there is a need for an organic catalyst that can provide the combined benefits of formulation flexibility, low water temperature bleaching performance and enzyme compatibility.

A process of producing 1-(4,4-dimethyl-3,4-dihydroisochinoline)decane-2-sulfate, which is known to be a bleach booster, is described in WO 01/16273:
1,2-decanediol is dissolved in carbon tetrachloride. Thionyl chloride is added drop wise at room temperature and the reaction mixture is heated to 60 °C. After some h time the reaction mixture is cooled using an ice bath. Water and acetonitrile are added as well as ruthenium chloride hydrate and sodium periodate. After stirring for an hour at room temperature, the reaction mixture is extracted with diethylether (4 times); the organic layers are subsequently washed with water (5 times), saturated sodium bicarbonate (3 times), brine (2 times), filtered through celite/silica gel, and dried over magnesium sulphate. After that the resulting liquid is concentrated to yield a clear oil, which oil is 1,2-decanediol cyclic sulphate. In the next reaction step 4,4-dimethyl-3,4-dihydroisoquinoline and acetonitrile are combined with the 1,2-decanediol cyclic sulphate, which is added in one portion. After another addition of acetonitrile the reaction mixture is stirred for some h. Then the precipitate is collected, washed with acetone and allowed to dry to give 1-(4,4-dimethyl-3,4-dihydroisochinoline)decane-2-sulfate.

While this process can be used in laboratory scale there exists a strong need to have a process that is usable in industrial scale, i.e. a process, which avoids the use of expensive educts such as thionyl chloride. Therefore it is one goal of the present invention to find a process, which avoids the use of thionyl chloride.

Another type of process to give a bleach booster is known from WO 03/104199. According to Example 4 of said patent application a sulphuric acid mono-[2-(3,4-dihydroisoquinoline-2-yl)-1-(2-ethyl-hexyloxymethyl)-ethyl ester is accessible by mixing cyclic sulphate, toluene and 3,4-dihydroisoquinoline in a flask and maintaining this mixture at a temperature of about 20 to 25 °C while stirring for 48 h. One ends up with the desired product in a yield of about 50 % in the form of a solid/gel, which needs to be filtered.

This process leading to another type of bleach booster is also not suitable for production in a large scale since the yield is much too low; and handling of a gel causes problems - especially during filtrations.

Therefore the goal of the present invention is not just to find a process of producing bleach boosters, which process avoids the use of thionyl chloride, but it is also a goal to find a process of producing bleach boosters, which process gives high yields. In addition to that the use of solvents like toluene in the last step of the reaction should be avoided, since aromatic solvents in consumer products are coming up for discussion and therefore should be avoided in a late state of synthesis because residues of them might be detectable in the end product, which end product can be used by an end-consumer. It is another goal of the present invention to find new compounds, which are accessible using this process and which compounds can be used as bleach boosters. The bleach boosters to be found should show about the same bleaching performance as known systems but should have a better dye-safety.

Surprisingly it has been found that these needs are meet by the process and the compounds according to the claims.

As used herein, the term "cleaning composition" includes, unless otherwise indicated, granular or powder-form all-purpose or "heavy-duty" washing agents, especially laundry detergents; liquid, gel or paste-form all-purpose washing agents, especially the so-called heavy-duty liquid types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, including antibacterial hand-wash types, laundry bars, mouthwashes, denture cleaners, car or carpet shampoos, bathroom cleaners; hair shampoos and hair-rinses; shower gels and foam baths and metal cleaners; as well as cleaning auxiliaries such as bleach additives and "stain-stick" or pre-treat types.

As used herein, the phrase "is independently selected from the group consisting of ....." means that moieties or elements that are selected from the referenced Markush group can be the same, can be different or any mixture of elements.

The test methods disclosed in the Test Methods Section of the present application must be used to determine the respective values of the parameters of Applicants' invention.

Unless otherwise noted, all component or composition levels are in reference to the active level of that component or composition, and are exclusive of impurities, for example, residual solvents or by-products, which may be present in commercially available sources.

All percentages and ratios are calculated by weight unless otherwise indicated. All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

A process of producing chemical compounds of formula I 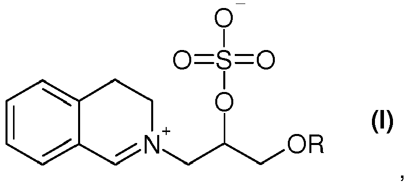 wherein R is alkyl, comprising at least one of the following steps:
a) producing dihydroisoquinoline from isoquinoline,
b) optionally producing a glycidylether from an alkohol and an epichlorhydrine,
c) reacting said dihydroisoquinoline with SO₃ and said glycidylether
is one of the cardinal aspects of the present invention.

This process can be used just performing step c), but it is also possible to perform steps a) and c); as well as a), b) and c). Processes in which two steps are used are preferred and a process in which all three steps are used is particularly preferred.

Suitable organic catalysts can be produced using a variety of reaction vessels and processes including batch, semi-batch and continuous processes.

In one aspect of Applicants invention, the process of making the aforementioned catalyst comprises the step of reacting 3,4-dihydroisoquinoline sulfur trioxide complex with an epoxide to form said organic catalyst.

In another aspect of Applicants' invention, the process of making the aforementioned catalyst comprises the steps of reacting 3,4-dihydroisoquinoline with a material selected from the group consisting of sulfur trioxide, a material that provides sulfur trioxide and mixtures thereof, to form a 3,4-dihydroisoquinoline sulfur trioxide complex, and reacting such 3,4-dihydroisoquinoline sulfur trioxide complex with an epoxide to form said organic catalyst.

In another aspect of Applicants' invention, the process of making the aforementioned catalyst comprises the step of reacting 3,4-dihydroisoquinoline with an epoxide sulfur trioxide complex to form said organic catalyst.

In another aspect of Applicants' invention, the process of making the aforementioned catalyst comprises the steps of reacting an epoxide with a material selected from the group consisting of sulfur trioxide, a material that provides sulfur trioxide and mixtures thereof, to form an epoxide sulfur trioxide complex, and reacting such epoxide sulfur trioxide complex with 3,4-dihydroisoquinoline to form said organic catalyst.

The oxaziridinium ring containing version of the aforementioned catalyst may be produced by contacting an iminium ring containing version of said catalyst with an oxygen transfer agent such as a peroxycarboxylic acid or a peroxymonosulfuric acid, for example, Oxone ®. Such species can be formed in situ and used without purification.

While the skilled artisan who processes the teachings of this specification can easily determine the desired reaction conditions and reactant concentrations, typical reaction parameters for the aforementioned aspects of Applicants' invention include reaction temperatures of from about 0 °C to about 150 °C, or from about 0 °C to about 125 °C, reaction pressures of from about 0.1 to about 100 atmospheres, from about 0.3 atmospheres to about 10 atmospheres or from about 1 atmosphere to about 10 atmospheres; reaction times of 0.1 hours to about 96 hours, from about 1 hour to about 72 hours, or from about 1 hour to about 24 hours. The reaction may also be run under an inert atmosphere or otherwise anhydrous conditions including, when a solvent is employed, the use of an anhydrous solvent.

Materials that are employed in practicing Applicants' process include 3,4-dihydroisoquinoline; epoxides and mixtures thereof; sulfur trioxide, sources of sulfur trioxide and mixtures thereof; and solvents.

When 3,4-dihydroisoquinoline is employed, the initial reaction mixture typically comprises from about 0.5 weight% to about 70 weight%, from about 5 weight% to about 70 weight%, or from about 10 weight% to about 50 weight% of such material. 3,4-Dihydroisoquinoline can be made according to the protocol found in Example 1.

When epoxides are employed, the initial reaction mixture typically comprises from about 0.5 weight% to about 70 weight%, from about 5 weight% to about 70 weight%, or from about 10 weight% to about 50 weight% of such material. Suitable epoxides include but are not limited to epoxides such as 2-propylheptyl glycidyl ether; 2-butyloctyl glycidyl ether; 2-pentylnonyl glycidyl ether; 2-hexyldecyl glycidyl ether; n-dodecyl glycidyl ether; n-tetradecyl glycidyl ether; n-hexadecyl glycidyl ether; n-octadecyl glycidyl ether; iso-nonyl glycidyl ether; iso-decyl glycidyl ether; iso-tridecyl glycidyl ether, and mixtures thereof. Such materials may contain oligomeric forms of the glycidyl ether which may optionally be removed prior to being employed as a reactant. 2-Propylheptyl glycidyl ether can be prepared as described in Example 2 of this specification. All of the other aforementioned glycidyl ethers can be prepared by following the generic protocol of Example 2 by substituting the appropriate alcohol in place of 2-propylheptanol. Suitable alcohols include 2-propylheptanol, 2-butyloctanol, 2-pentylnonanol, 2-hexyldecanol, n-dodecanol, n-tetradecanol, n-hexadecanol, n-octadecanol, iso-nonanol, iso-decanol and iso-tridecanol.

When sulfur trioxide, sources of sulfur trioxide and mixtures thereof are employed, the initial reaction mixture typically comprises from about 0.5 weight% to about 70 weight%, from about 5 weight% to about 70 weight%, or from about 10 weight% to about 50 weight% of such material. Suitable materials include sulfur trioxide, and sulfur trioxide complexes such as sulfur trioxide trimethylamine, sulfur trioxide dioxane, sulfur trioxide pyridine, sulfur trioxide N,N-dimethylformamide, sulfur trioxide sulfolane, sulfur trioxide tetrahydrofuran, sulfur trioxide diethylether, sulfur trioxide 3,4-dihydroisoquinoline and mixtures thereof.

The balance of any reaction mixture is typically solvent. When a solvent is employed, the initial reaction mixture typically comprises up to 99 weight% solvent, from about 10 weight% to about 90 weight% solvent, or from about 20 weight% to about 80 weight% solvent. Suitable solvents include aprotic, polar and apolar solvents such as acetonitile, dioxane, tertbutyl methylether, tetrahydrofuran, N,N-dimethylformamide, sulfolane, chlorobenzene, toluene, 1,2 -dichloroethane, methylene chloride, chloroform, diethyl ether, hexanes, pentanes, benzene, xylenes and mixtures thereof. Suitable solvents can be purchased from Aldrich, P.O. Box 2060, Milwaukee, WI 53201, USA.

The present invention relates to a process as described above and comprising step a) where the dihydroisoquinoline in step a) is produced by
ai) reducing an isoquinoline to give a tetrahydroisoquinoline and
aii) oxidizing said tetrahydroisoquinoline to give the dihydroisoquinoline.

This process provides the best results in case the oxidation in step aii) is performed using sodium hypochloride and/or potassium hypochloride, as is suggested for such reactions in DE 195 07 552 A1, since the exceeding oxidation of the product to isoquinoline is substantially suppressed.

It is even more preferred to have a process as described above, wherein step a) further comprises steps
aiii) extracting the dihydroisoquinoline obtained in aii) with an organic solvent
aiv) distilling the product obtained in aiii).

It is possible to extract the dihydroisoquinoline with all kinds of organic solvents, which solvents have to match the following criteria: They have to be substantially insoluble in water, need to be acceptable solvents for the dihydroisoquinoline to be extracted and should form an azeotrope with water. Examples of such solvents are benzene, toluene, xylene, with toluene being preferred.

None the less it is also possible to perform the process as described above without using a solvent that fulfils all criteria; e.g. it is also possible to use a solvent that does not form an azeotrope with water. In such a case the extraction step itself could be performed in the same way. However, it is preferred to use a solvent, which forms an azeotrope with water since by doing this the water used during the reaction can be drawn off easily by codistillation. This aspect of codistillation is crucial for large scale applications, since the distillation of water is quite expensive due to the high boiling enthalphie of water.

Other methods of purifying the product of step a) as are mentioned in step aiv) are also possible and lie within the scope of the present invention. Such methods are e.g. crystallisation or chromatography. Purification by distillation is most preferred, however, it is also possible to use the rare product as educt for the synthesis to follow.

Two other routes to produce the dihydroisoquinoline are possible and lie within the scope of the present invention:
ax): Dihydroisoquinoline can be produced using a Bischler-Napieralski reaction: 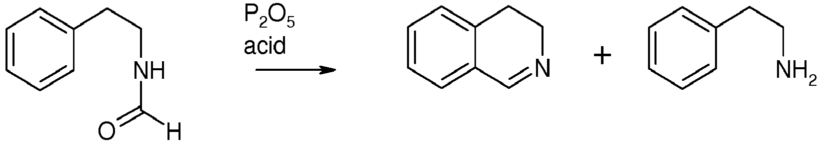
A process as described above, wherein step ax) is performed using phosphorous pentoxide and an acid is another preferred object of the present invention, whereby it is preferred when the acid is selected from the group consisting of poly phosphorous acid, trifluormethaneacid, formic acid and methane sulfonic acid. Compared to standard Bischler-Napieralski reaction conditions the amount of phosphor-containing compounds as well as the reaction time could be reduced. This makes the reaction less expensive and has a positive effect with respect to the overall environmental rating of the process. Instead of poly phosphorous acid (PPA) methanesulfonic acid (MSA) can be used. This is not possible when using MSA alone. At temperatures of about 160 °C, which is above the decomposition temperature of MSA, which is about 140 °C, good results can be obtained.

It is further preferred when in the process as described above the reaction mixture of step ax) is neutralized using KOH, because this leads to salts that have a higher solubility.

A process as described above, wherein after neutralization the amine is oxidized to give an imine using sodium hypochlorite forms another preferred embodiment of the present invention.

An alternative for this route is ay): 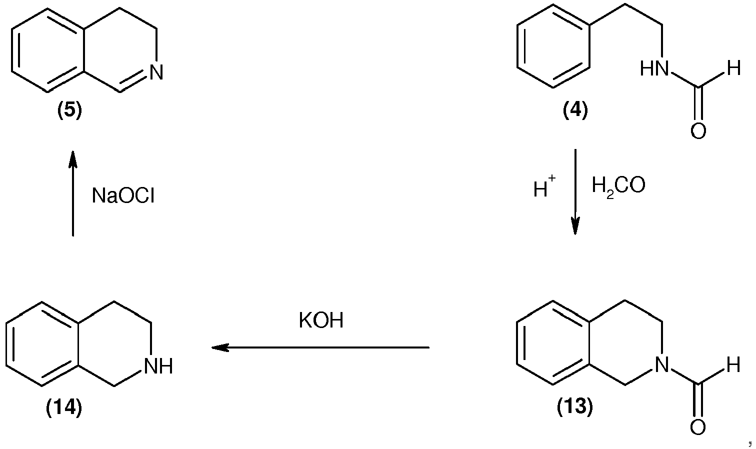 to perform a Pictet-Spengler reaction from (4) to (13), followed by cleavage of the amide to give (14) and subsequent oxidation using sodium hypochlorite. A great advantage of this alternative is to be seen in the fact that the reaction can be performed as a one pot reaction. An additional advantage is the fact, that this is a phosphate free route to obtain the desired products, which means lower cost for waste disposal and an environmentally friendly process.

As a source of formaldehyde trioxane is advantageous, since it has a melting point of 62 °C, and therefore can be applied easily as a liquid. It is obvious that a heated feed pipe can advantageously been used. As the acid all kinds of strong acids, such as trifluoracetic acid, formic acid or methane sulfonic acid can be used.

A process as described above and comprising step b) is also particularly preferred in case the glycidylether in step b) is produced by
bia) addition of epichlorohydrine to alcohol in the presence of a lewis acetic catalyst and subsequent reaction of the resulting chlorohydrine with NaOH and/or KOH or
bib) reacting epichlorohydrine with an alcohol in the presence of a phase transfer catalyst together with NaOH and/or KOH.

A process as described above, wherein the glycidylether obtained in step bia) and/or bib) is purified by distillation forms a particularly preferred embodiment of the present invention. As has already been discussed above with respect to step a) distillation is the most preferred method of purification, i.e. other methods of purification such as crystallisation or chromatography are also possible and lie within the scope of the present invention.

According to the present invention, step c) comprises the following steps:
ci) dissolving dihydroisochinoline in a solvent,
cii) adding SO₃ to the solution obtained in ci), wherein SO₃ is used in an excess with respect to dihydroisochinoline,
ciii) adding glycidylether to the solution obtained in cii),
civ) heating the mixture obtained in ciii),
cv) quenching remaining SO₃ in the mixture obtained in ciii) or civ), ,
cvii) inducing crystallisation,
cviii) filtering the crystals off the mixture obtained in cvii),
cix) purifying the crystals obtained in cviii),
cx) drying the crystals obtained in cviii) or cix),
   optionally further comprising step
cvi) exchanging a substantial part of the solvent of the mixture obtained in ciii), civ), or cv).

Preferred embodiments of the present invention are those processes as described above, wherein:
α) the solvent in step ci) is inert with respect to SO₃ and/or
χ) step cii) takes place at a temperature of 0 °C or above and/or
δ) the glycidylether in step ciii) is used in an excess with respect to dihydroisoquinoline and/or
ε) step ciii) takes place at a temperature of 0 °C or above and/or
φ) the SO₃ in step cii) is used in greater excess with respect to the dihydroisoquinoline than is the glycidylether in step ciii) and/or
γ) heating in step civ) is performed under reflux and/or
η) the quenching in step cv) is performed using a base and/or
ηa) the amount of base used according to η) exactly matches the surplus of SO₃ or exeeds the surplus of SO₃ and/or
τ) at least 50 % of the solvent of the mixture in step cv) are exchanged and/or
ϕ) the solvent that is added in step cvi) is
   an alcohol or
   a mixture of alcohols or
   a mixture of one or more alcohols with one or more polar aprotic solvent(s) and/or
κ) crystallization in step cvii) is induced by decreasing the temperature using a temperature ramp having zero, one or more plateaus and/or
λ) crystallization in step cvii) is induced by decreasing the temperature using a temperature ramp with the temperature being decreased at a rate of 1 to 20 °C / h.

A process as described above, wherein:
α') the solvent in step ci) is dichloroethane or dioxane or a mixture of both and/or
β') the SO₃ in step cii) is used in an amount of 1.05 to 1.15 mol per mol of dihydroisoquinoline and/or
χ') step cii) takes place at a temperature of 29 °C or above and/or
δ') the glycidylether in step ciii) is used in an amount of 1.01 to 1.11 mol per mol of dihydroisoquinoline and/or
ε') step cii) takes place at a temperature of 29 °C or above and/or
φ') the ratio of the excess of SO₃ in step cii) to the excess of the glycidylether in step ciii) - both with respect to the dihydroisoquinoline - is in the range of 1,01 - 10 : 1 and/or
γ') the temperature in step civ) is 60 °C or above and/or
η') the quenching in step cv) is performed using one or more selected from the group consisting of amines, dihydroisoquinoline, NaOH, KOH and/or aminic base and/or
η'a) the amount of KOH and/or NaOH and/or amine used according to η') exactly matches the surplus of SO₃ or exeeds the surplus of SO₃ and/or
τ') at least 80 % of the solvent of the mixture in step cv) are exchanged and/or
ϕ') the alcoholic solvent that is added in step cvi) is EtOH, MeOH or iPrOH,
   the mixture of alcohols comprises at least one of EtOH, MeOH or iPrOH or
   the mixture of alcohol(s) and polar aprotic solvent comprises acetic acid ester as the polar aprotic solvent component
   and/or
κ') crystallization in step cvii) is induced by decreasing the temperature using a temperature ramp having three plateaus and/or
λ') crystallization in step cvii) is induced by decreasing the temperature using a temperature ramp with the temperature being decreased at a rate / at rates of 5 to 10 °C/h
forms an even more preferred embodiment of the present invention.

To receive good results using the processes described above, it is preferred, to dissolve dihydroisochinoline in a solvent, which solvent in step ci) is inert with respect to SO₃. An embodiment in which the solvent in step ci) is dichloroethane is most preferred. Also dioxane can be used giving good results, - the same is true for mixtures of these solvents. The use of dichloroethane is advantageous compared to the use of other solvents such as e.g. acetonitrile, which is described in the state of the art. Acetonitrile as well as e.g. propionitrile, butyronitrile, THF, dibuthylether and acetic acid ester are not inert against SO₃. This is important since the use of SO₃ is desired to avoid the more expensive thionyl chloride (see below). This means that it is possible to run the process using such solvents but the yields are quite low (about 45 %). Other solvents such as dichloromethane are also not inert with respect to SO₃ and in addition to that have a boiling point, which is quite low. Diglyme, glyme, toluene, chlorobenzene, N-methylpyrrolidone (NMP) or propylenecarbonate also lead to low yields, when used in combination with SO₃-complexes formed *in situ.*

In step cii) it is preferred that SOsis added to the solution obtained in ci). This supersedes the need to laboriously handle isolated expensive SO₃-complexes. The SO₃ is added as a pure substance, i.e. freshly distilled or stabilised. This leads to an unexpected advantage with respect to the purity of the product. It was found that in case of the use of SO₃-amine-complexes the amines, e.g. NMe₃, tend to react with the glycidylether and that in case of the use of SO₃-dioxane-complexes dioxane is incorporated in the product. Therefore the use of SO₃ is not only easier and cheaper than the use of complexes but has the additional advantage of leading to products, which contain less impurities. The yield of the product can be further increased by using the SO₃ in excess with respect to dihydroisoquinoline and the best results were obtained in case the SO₃ is used in an amount of 1.05 to 1.15 mol per mol of dihydroisoquinoline. The temperature in this step cii) can be -20 °C, preferably 0 °C or above, it is preferred that the temperature is 29 °C or above. In general temperatures from about -10 °C to the boiling point of the solvent can be used, a temperature in the range of about 0 to about 40 °C is preferred, a temperature in the range of about 20 to about 35 °C is more preferred, a temperature in the range of from about 25 to about 32 °C is even more preferred, and most preferred is a temperature of about 30 °C, such as e.g. 28, 29, 30 or 31 °C.

As the next step glycidylether is added to the solution obtained in cii), whereby it is preferred that the glycidylether is used in an excess with respect to dihydroisoquinoline. In a preferred embodiment of the present invention the glycidylether is used in an amount of 1.01 to 1.1 mol per mol of dihydroisoquinoline. The temperature in this step ciii) can be -20 °C, preferably 0 °C or above, it is preferred that the temperature is 29 °C or above. In general temperatures from about -10 ° to the boiling point of the solvent can be used, a temperature in the range of about 0 to about 40 °C is preferred, a temperature in the range of about 20 to about 35 °C is more preferred, a temperature in the range of from about 25 to about 32 °C is even more preferred, and most preferred is a temperature of about 30 °C, such as e.g. 28, 29, 30 or 31 °C.

In a particularly preferred embodiment of the present invention the ratio of the excess of SO₃ in step cii) to the excess of the glycidylether in step ciii) - both with respect to the dihydroisoquinoline - is in the range of 1.01 - 10 : 1. In a case in which 1.0 eq. of dihydroisoquinoline, 1.1 eq. of SO₃ and 1.05 eq. of glycidylether are used the excess of SO₃ is 0.1, the excess of glycidylether is 0.05 and therefore the ratio would be 2 : 1. Preferred are ratios from 1.1 to 5 : 1, even more preferred are ratios from 1.5 to 3 : 1.

Next the reaction mixture obtained in ciii) can be heated, with heating being preferred. A preferred embodiment of the present invention is a process in which heating in step civ) is performed under reflux and an even more preferred embodiment is a process with the temperature being 60 °C or above.

Subsequently the remaining SO₃ in the mixture obtained in ciii) or civ) is quenched, whereby the quenching in step cv) preferably is performed using a base and more preferably is performed using one or more selected from the group consisting of amines, dihydroisoquinoline, isoquinoline, NaOH and KOH.

When quenching the surplus of SO₃ there exist two preferred procedures:

One is to perform the quenching step cv) with an amount of base that matches the surplus of SO₃. Such a procedure has the advantage, that the minimal amount of base, that is needed for quenching is used, which keeps the costs low in two ways: first by reducing the amount of base used and second by yielding a waste product that does not need to be neutralized. Therefore this procedure also is advantageous from an environmental point of view.

The other is to perform the quenching step cv) with a surplus of base with respect to the surplus of SO₃. This procedure has the advantage, that it suppresses the development of acidic compounds, such as sulphuric acid or sulphuric acid semi ester. Those acidic compounds lead to a decomposition of the sulphate group in the end product. During such decomposition another acidic species forms, which means that this process is autocatalytic and it is the reason for a reduced practical storage life. Therefore by using a surplus of base with respect to the surplus of SO₃ in quenching step cv) one will end up with an end product that is free flowing and does not agglutinate when stored over longer periods.

Exchanging a substantial part of the solvent of the mixture obtained in ciii), civ) or cv) is another preferred embodiment of the present invention. Thereby it is preferred when at least 50 %, more preferred at least 80 % and mostly preferred at least 90 % of the solvent of the mixture in step cv) are exchanged. It is preferred that the solvent that is added in this step is an alcohol, a mixture of alcohols or a mixture of one or more alcohols with one or more polar aprotic solvent(s), whereby in the most preferred embodiments of the present invention the alcoholic solvent that is added in step cvi) is EtOH, MeOH or iPrOH, the mixture of alcohols comprises at least one of EtOH, MeOH or iPrOH or the mixture of alcohol(s) and polar aprotic solvent comprises acetic acid ester as the polar aprotic solvent component. To reduce the amount of solvent which is used for the exchange said exchange can not only be performed in one step but also in more than one step, e.g. in two, three, four or more steps. An exchange of a substantial part of the solvent is preferred, however, it is also possible to continue without an exchange.

Next crystallisation is induced. This can be done in a number of ways, e.g. by decreasing the temperature, by distilling off the solvent, at reduced pressure - where advantageous, or by adding solvents, which reduce the solubility of the product in the solvent-mixture. It is preferred to induce crystallisation in step cvii) by decreasing the temperature. Using a temperature ramp is more preferred. This temperature ramp preferably has zero, one or more plateaus whereby it is mostly preferred if it has three plateaus. A plateau in the sense of the present invention is a period during which the temperature does not decrease or does decrease at a rate, which is significantly, i.e. by a factor of at least 5, lower than the rate-average during the cooling periods. Has the mixture been cooled from e.g. 80 to 60 °C at a rate of 10 °C / h and is than cooled for another h at a rate of e.g. 1 °C / h, this second period would be called a plateau. This term is also used in case no further decrease in temperature occurs before the mixture is filtered.

Inducing the crystallization in step cvii) by decreasing the temperature using a temperature ramp with the temperature being decreased at a rate of 1 to 20 °C / h is preferred. Inducing the crystallization in step cvii) by decreasing the temperature using a temperature ramp with the temperature being decreased at a rate / at rates of 5 to 10 °C / h is even more preferred.

The use of seed crystals to fasten the process and/or to tailor the crystal size, form and/or modification lies within the scope of the present invention and forms a preferred embodiment.

The process as described above can be performed as a continuous process as well as a process having separate steps. In such a case each of these steps can be performed as batch or semi-batch process or as a continuous process. The reaction can be performed under normal conditions, i.e. under atmospheric pressure, however, positive pressure is also possible during the process. During distillation steps positive pressure as well as atmospheric pressure can be used, however, distillation under reduced pressure is advantageous. The temperatures during the reaction in general range from - 40 to 200 °C, in particular from - 10 to 100 °C unless otherwise noted. Inert gas can be used in any step to protect the products or to aid distillation.

The present invention is also directed to a compound, which is producible by a process as described above and of course to a compound, which is produced by a process as described above. This does also include mixtures of such compounds independent on whether they were produced by performing the reaction using two or more different starting materials or by performing two or more reactions - one with each of the two or more different starting materials alone - and than mixing the end products.

The present invention is directed to a compound according to formula I 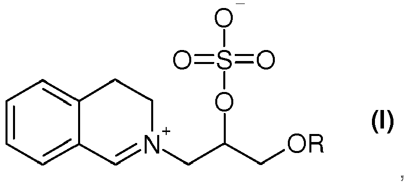 wherein R is selected from the group consisting of 2-butyloctyl, tridecanyl, 2-propylheptyl, 2-pentylnonanyl and 2-hexyldecyl.

Those compounds wherein R in formula I above is a branched alkyl are preferred and those wherein R is a saturated alkyl are more preferred. Most preferred is a compound with R being a branched saturated alkyl. A preferred compound is one of formula I above, wherein R is a group having 9 to 24 C-atoms, such as 9, 10, 11, 12, 13,...or 24 C-atoms, preferably 12 to 20 C-atoms and even more preferred 12 to 18 C-atoms. More precisely R of formula I preferably is a branched alkyl group containing from 9 to 24 carbons or a linear alkyl group containing from 11 to 24 carbons. Compounds wherein R in formula I above is a branched alkyl group containing from 9 to 18 carbons or a linear alkyl group containing from 11 to 18 carbons are even more preferred.

Therefore a compound as mentioned above, wherein R of formula I is selected from the group consisting of 2-propylheptyl, 2-butyloctyl, 2-pentylnonyl, 2-hexyldecyl, n-dodecyl, n-tetradecyl, n-hexadecyl, n-octadecyl, iso-nonyl, iso-decyl, iso-tridecyl and iso-pentadecyl forms a preferred embodiment of the present invention. And a compound wherein R of formula I is selected from the group consisting of 2-butyloctyl, tridekanyl, 2-propylheptyl, 2-pentylnonanyl, 2-hexyldecyl, iso-tridecyl and iso-pentadecyl forms a particularly preferred embodiment of the present invention. Most preferred is a compound wherein R is 2-butyloctyl.

Applicants have found that the organic catalyst of the present invention results in improved enzyme compatibility. While not being bound by theory, Applicants believe this is due to favourable partitioning of the catalyst in aqueous environments.

In one aspect of Applicants' invention, Applicants' organic catalyst has an enzyme compatibility value of 70 or greater, or even 80 or greater.

These compounds can be used as components in all kinds of cleaning compositions, which includes granular or powder-form all-purpose or "heavy-duty" washing agents, especially laundry detergents; liquid, gel or paste-form all-purpose washing agents, especially the so-called heavy-duty liquid types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, including antibacterial hand-wash types, laundry bars, mouthwashes, denture cleaners, car or carpet shampoos, bathroom cleaners; hair shampoos and hair-rinses; shower gels and foam baths and metal cleaners; as well as cleaning auxiliaries such as bleach additives and "stain-stick" or pre-treat types.

Surprisingly it was found that the compounds of the present invention lead to better low water temperature bleaching performance, when used in a cleaning composition. In addition to that the unexpected effect of good enzyme compatibility was found. Typical enzymes that are used in cleaning compositions include, but are not limited to, hemicellulases, peroxidases, proteases, cellulases, xylanases, lipases, phospholipases, esterases, cutinases, pectinases, mannanases, pectate lyases, keratinases, reductases, oxidases, phenoloxidases, lipoxygenases, ligninases, pullulanases, tannases, pentosanases, malanases, β-glucanases, arabinosidases, hyaluronidase, chondroitinase, laccase, and amylases, or mixtures thereof.

Cleaning compositions may be advantageously employed for example, in laundry applications, hard surface cleaning, automatic dishwashing applications, as well as cosmetic applications such as dentures, teeth, hair and skin. However, due to the unique advantages of both increased effectiveness in lower temperature solutions and the superior enzyme compatiblity, the organic catalysts of the present invention are ideally suited for laundry applications such as the bleaching of fabrics through the use of bleach containing detergents or laundry bleach additives. Furthermore, the organic catalysts of the present invention may be employed in both granular and liquid compositions.

The organic catalysts of the present invention may also be employed in a cleaning additive product. A cleaning additive product including the organic catalysts of the present invention is ideally suited for inclusion in a wash process when additional bleaching effectiveness is desired. Such instances may include but, are not limited to, low temperature solution cleaning application. The additive product may be, in its simplest form, Applicants' organic catalyst. Preferably, the additive could be packaged in dosage form for addition to a cleaning process where a source of peroxygen is employed and increased bleaching effectiveness is desired. Such single dosage form may comprise a pill, tablet, gelcap or other single dosage unit such as pre-measured powders or liquids. A filler or carrier material may be included to increase the volume of such composition. Suitable filler or carrier materials include, but are not limited to, various salts of sulfate, carbonate and silicate as well as talc or clay. Filler or carrier materials for liquid compositions may be water or low molecular weight primary and secondary alcohols including polyols and diols. Examples of such alcohols include, but are not limited to, methanol, ethanol, propanol and isopropanol. The compositions may contain from about 5 % to about 90 % of such materials. Acidic fillers can be used to reduce pH. Alternatively, the cleaning additive may include an activated peroxygen source defined below or the adjunct ingredients as fully defined below.

Applicants' cleaning compositions and cleaning additives require a catalytically effective amount of Applicants' organic catalyst. The required level of such catalyst may be achieved by the addition of one or more species of Applicants' organic catalyst. As a practical matter, and not by way of limitation, the compositions and cleaning processes herein can be adjusted to provide on the order of at least 0.001 ppm, from about 0.001 ppm to about 500 ppm, from about 0.005 ppm to about 150 ppm, or even from about 0.05 ppm to about 50 ppm of Applicants' organic catalyst in the wash liquor. In order to obtain such levels in the wash liquor, typical compositions herein may comprise from about 0.0002% to about 5%, or even from about 0.001 % to about 1.5%, of organic catalyst, by weight of the cleaning compositions.

When the Applicants' organic catalyst is employed in a granular composition, it may be desirable for the Applicants' organic catalyst to be in the form of an encapsulated particle to protect the Applicants' organic catalyst from moisture and/or other components of the granular composition during storage. In addition, encapsulation is also a means of controlling the availability of the Applicants' organic catalyst during the cleaning process and may enhance the bleaching performance of the Applicants' organic catalyst. In this regard, the Applicants' organic catalyst can be encapsulated with any encapsulating material known in the art.

The encapsulating material typically encapsulates at least part, preferably all, of the Applicants' organic catalyst. Typically, the encapsulating material is water-soluble and/or water-dispersible. The encapsulating material may have a glass transition temperature (Tg) of 0 °C or higher.

The encapsulating material is preferably selected from the group consisting of carbohydrates, natural or synthetic gums, chitin and chitosan, cellulose and cellulose derivatives, silicates, phosphates, borates, polyvinyl alcohol, polyethylene glycol, paraffin waxes and combinations thereof. Preferably the encapsulating material is a carbohydrate, typically selected from the group consisting of monosaccharides, oligosaccharides, polysaccharides, and combinations thereof. Most preferably, the encapsulating material is a starch. Preferred starches are described in EP 0 922 499; U.S. 4,977,252; U.S. 5,354,559 and U.S. 5,935,826.

The encapsulating material may be a microsphere made from plastic such as thermoplastics, acrylonitrile, methacrylonitrile, polyacrylonitrile, polymethacrylonitrile and mixtures thereof; commercially available microspheres that can be used are those supplied by Expancel of Stockviksverken, Sweden under the trademark Expancel®, and those supplied by PQ Corp. of Valley Forge, Pennsylvania USA under the tradename PM 6545, PM 6550, PM 7220, PM 7228, Extendospheres®, Luxsil®, Q-cel® and Sphericel®.

The cleaning compositions herein will preferably be formulated such that, during use in aqueous cleaning operations, the wash water will have a pH of between about 6.5 and about 11, or even about 7.5 and 10.5. Liquid dishwashing product formulations may have a pH between about 6.8 and about 9.0. Laundry products typically have a pH of from about 9 to about 11. Techniques for controlling pH at recommended usage levels include the use of buffers, alkalis, acids, etc., and are well known to those skilled in the art.

### Adjunct Materials

While not essential for the purposes of the present invention, the non-limiting list of adjuncts illustrated hereinafter are suitable for use in the instant compositions and may be desirably incorporated in certain embodiments of the invention, for example to assist or enhance cleaning performance, for treatment of the substrate to be cleaned, or to modify the aesthetics of the cleaning composition as is the case with perfumes, colorants, or dyes. The precise nature of these additional components, and levels of incorporation thereof, will depend on the physical form of the composition and the nature of the cleaning operation for which it is to be used. Suitable adjunct materials include, but are not limited to, surfactants, builders, chelating agents, dye transfer inhibiting agents, dispersants, enzymes, and enzyme stabilizers, catalytic materials, bleach activators, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, hydrotropes, processing aids, solvents and/or pigments. In addition to the disclosure below, suitable examples of such other adjuncts and levels of use are found in U.S. Patent Nos. 5,576,282, 6,306,812 B1 and 6,326,348 B1 that are incorporated by reference.

The cleaning compositions can be formulated into any suitable form and prepared by any process chosen by the formulator, non-limiting examples of which are described in Applicants' examples and in U.S. 5,879,584; U.S. 5,691,297; U.S. 5,574,005; U.S. 5,569,645; U.S. 5,565,422; U.S. 5,516,448; U.S. 5,489,392; U.S. 5,486,303 all of which are incorporated herein by reference.

### Organic Catalyst/Enzyme Compatibility Test:

The test described below uses an alpha amylase activity assay to measure the impact of organic catalysts on the enzyme.

Equipment. UV/Vis spectrophotometer capable of measuring @ 415 nm, heated magnetic stirrer capable of 40 °C, 5 ml Luer lock syringe and filters (Acrodisc 0.45 · µm), pH meter, and balance (4-place analytical).
Reagents. Merck Amylase Kit (Merck Eurolab, Cat. No. 1.19718.0001); Trizma Base (Sigma Cat # T-1503, or equivalent); Calcium Chloride Dihydrate (Sigma Cat # C-5080, or equivalent); Sodium Thiosulfate Pentahydrate (Sigma Cat # S-6672 or equivalent); Hydrochloric Acid (VWR Cat # JT9535-0, or equivalent); Hardness solution (CTC Group, 3.00 gr/cc or equivalent); Sodium Percarbonate; Peracetic Acid (Aldrich, Cat.# 26933-6 or equivalent); Amylase enzymes: Termamyl, Natalase, and Duramyl (Novozymes, Denmark); Granular detergent matrix containing no enzyme, organic catalyst or bleaching agents.

### 1.) Solution Preparation: prepare the following:

a) TRIS Assay Buffer. Prepare 1 liter of 0.1 M TRIS buffer, 0.5% sodium thiosulphate (W/V), 0.11 % calcium chloride (w/v) at pH 8.3.
b) Blank Detergent Solution. Prepare one liter of 0.5% enzyme and bleach free granular detergent product in deionized water (W/V) that is 250 ppm H₂O₂ (0.77 gm percarbonate) and 10 gpg hardness (880 UI of hardness).
c) Termamyl, Duramyl and Natalase Stock. Make 100 ml solutions each of a 0.1633 mg active Termamyl per ml TRIS Buffer, a 0.1159 mg active Natalase per ml TRIS Buffer, and a 0.1596 mg active Duramyl per ml TRIS Buffer.
d) Organic catalyst stocks. Make a 500 ppm in methanol solution of µm.
e) Peracetic acid stock. Make a 3955 ppm peracetic acid solution in deionized water.
f) Amylase reagent. Follow Merck kit instructions for preparing flacons (containers) 1 and 2 using flacon 3 and subsequent mixing of flacons 1 and 2 to produce the final reagent used in the amylase activity analysis.

### 2.) Sample Analysis:

a.) Analysis of sample with enzyme only: Add 100 ml of blank detergent solution to a 150 ml beaker. Place beaker on heated stir plate and bring temperature to 40 °C with stirring. Add Y µl of enzyme stock to the beaker where Y= 612 µl for Duramyl, 306 µl for Termamyl, or 918 µl for Natalase. Spike only enzyme of interest. Stir sample for 1 minute. Start timer. At 7 minutes 45 seconds, pull a sample and filter it using a 0.45 µm syringe filter (5 ml syringe). Mix 6 µl of filtered sample with 250 µl of amylase reagent in a cuvette and place the cuvette in a UV/VIS spectrophotometer and monitor change in absorbance at 415 nm. Determine length of time (tE) to the nearest second required to obtain an absorbance reading of 1.0 for each enzyme. Use each enzyme's tE in Steps 2.)b.) and 2.)c.) below.
b.) Analysis of sample with enzyme and peracetic acid only. Follow Step 2.)a.) except after enzyme addition, allow solution to stir for 1 minute then add 127 µl of peracetic acid stock and start timer. Pull sample at 7 minutes 45 seconds as in Step 2.)a.). Once sample and reagent are mixed, record the absorbance at tE for the respective enzyme. Designate such absorbance Ab.
c.) Analysis of sample with enzyme, peracetic acid, and organic catalyst. Follow Step 2.)a.) except after enzyme addition, allow solution to stir for 1 minute then add 127 µl of peracetic acid stock and 100 µl of organic catalyst stock and start timer. Pull sample at 7 minutes 45 seconds as in Step 2.)a.). Once sample and reagent are mixed, record the absorbance at tE for the respective enzyme. Designate such absorbance Ac.

### 3.) Calculate Enzyme Compatibility Value (ECV)

a.) Calculate the ECV for each specific enzyme: termamyl (ECVter), duramyl (ECVdur) and natalase (ECVnat). The ECV for any specific enzyme is (Ac/Ab) x 100 where Ab and Ac are the values determined in Steps 2.)b.) and 2.)c.), respectively, for that enzyme.
b.) The ECV for a given organic catalyst is the average of the individual ECV values for the three enzymes. Thus, ECV = (ECVter + ECVdur + ECVnat)/3.

### Examples:

The examples are divided into three general sections:
Section 1 (Examples 1-12) deals with the synthesis of different substances with the focus being on the different products,
Section 2 (Examples 13-56) is more directed to the different synthesis routes and
Section 3 (Example 57) shows the behaviour of the compounds when tested according to Applicants' Organic Catalyst/Enzyme Compatibility Test.

Unless otherwise indicated, materials can be obtained from Aldrich, P.O. Box 2060, Milwaukee, WI 53201, USA. In Examples 1 - 12, the solvent acetonitrile may be replaced with other solvents, including but not limited to, 1,2-dichloroethane.

### Section 1:

### Example 1:

### Preparation of Sulfuric acid mono-[2-(3,4-dihydro-isoquinolin-2-yl)-1-(2-propylheptyloxymethyl)-ethyl] ester, internal salt:

### Preparation of 2-propylheptyl glycidyl ether:

To a flame dried, 500 ml round bottomed flask equipped with an addition funnel charged with epichlorohydrin (15.62 g, 0.17 mol), is added 2-propylheptanol (Pfaltz & Bauer, Inc., 172 E. Aurora Street, Waterbury CT, 06708, USA) (20 g, 0.127 mol) and stannic chloride (0.20 g, 0.001 mol). The reaction is kept under an argon atmosphere and warmed to 90 °C using an oil bath. Epichlorohydrin is dripped into the stirring solution over 60 minutes followed by stirring at 90 °C for 18 hours. The reaction is fitted with a vacuum distillation head and 1-chloro-3-(2-propyl-heptyloxy)-propan-2-ol is distilled at a temperature range of 90 °C -> 95 °C under 0.2mm Hg. Wt.=22.1 g. The 1-chloro-3-(2-propyl-heptyloxy)-propan-2-ol (5.0 g, 0.020 mol) is dissolved in tetrahydrofuran (50 ml) and stirred at RT under an argon atmosphere. To the stirring solution is added potassium tert-butoxide (2.52 g, 0.022 mol) and the suspension is stirred at RT for 18 hours. The reaction is then evaporated to dryness, residue dissolved in hexanes and washed with water (100 ml). The hexanes phase is separated, dried with Na₂SO₄, filtered and evaporated to dryness to yield the crude 2-propylheptyl glycidyl ether, which can be further purified by vacuum distillation.

### Preparation of Sulfuric acid mono-[2-(3,4-dihydro-isoquinolin-2-yl)-1-(2-propylheptyloxymethyl)-ethyl] ester, internal salt:

To a flame dried 250 ml three neck round bottomed flask, equipped with a condenser, dry argon inlet, magnetic stir bar, thermometer, and heating bath is added 3,4-dihydroisoquinoline (0.38 mol.; prepared as described in Example I of U.S. 5,576,282), 2-propylheptyl glycidyl ether (0.38 mol, prepared as described above), SO₃-DMF complex (0.38 mol), and acetonitrile (500 ml). The reaction is warmed to 80 °C and stirred at temperature for 72 hours. The reaction is cooled to room temperature, evaporated to dryness and the residue recrystallized from ethyl acetate and/or ethanol to yield the desired product.

### Example 2:

### Preparation of Sulfuric acid mono-[2-(3,4-dihydro-isoquinolin-2-yl)-1-(2-butyl-octyloxymethyl)-ethyl] ester, internal salt:

The desired product is prepared according to Example 1, substituting 2-butyloctanol for 2-propylheptanol.

### Example 3:

### Preparation of Sulfuric acid mono-[2-(3,4-dihydro-isoquinolin-2-yl)-1-(2-pentyl-nonyloxymethyl)-ethyl] ester, internal salt:

The desired product is prepared according to Example 1, substituting 2-pentylnonanol (obtained from Pfaltz & Bauer, Inc., Wayerbury, CT 06708) for 2-propylheptanol.

### Example 4:

### Preparation of Sulfuric acid mono-[2-(3,4-dihydro-isoquinolin-2-yl)-1-(2-hexyl-decyloxymethyl)-ethyl] ester, internal salt:

The desired product is prepared according to Example 1, substituting 2-hexyldecanol for 2-propylheptanol.

### Example 5:

### Preparation of Sulfuric acid mono-[2-(3,4-dihydro-isoquinolin-2-yl)-1-(dodecyloxymethyl)-ethyl] ester, internal salt:

The desired product is prepared according to Example 1, substituting n-dodecanol for 2-propylheptanol.

### Example 6:

### Preparation of Sulfuric acid mono-[2-(3,4-dihydro-isoquinolin-2-yl)-1-(tetradecyloxymethyl)-ethyl] ester, internal salt:

The desired product is prepared according to Example 1, substituting n-tetradecanol for 2-propylheptanol.

### Example 7:

### Preparation of Sulfuric acid mono-[2-(3,4-dihydro-isoquinolin-2-yl)-1-(hexadecyloxymethyl)-ethyl] ester, internal salt:

The desired product is prepared according to Example 1, substituting n-hexadecanol for 2-propylheptanol.

### Example 8:

### Preparation of Sulfuric acid mono-[2-(3,4-dihydro-isoquinolin-2-yl)-1-(octadecyloxymethyl)-ethyl] ester, internal salt:

The desired product is prepared according to Example 1, substituting n-octadecanol for 2-propylheptanol.

### Example 9:

### Preparation of Sulfuric acid mono-[2-(3,4-dihydro-isoquinolin-2-yl)-1-(iso-nonyloxymethyl)-ethyl] ester, internal salt:

The desired product is prepared according to Example 1, substituting iso-nonanol (Exxal 9 obtained from Exxon Mobile Chemical, Houston, Texas USA) for 2-propylheptanol.

### Example 10:

### Preparation of Sulfuric acid mono-[2-(3,4-dihydro-isoquinolin-2-yl)-1-(iso-decyloxymethyl)-ethyl] ester, internal salt:

The desired product is prepared according to Example 1, substituting iso-decanol (obtained from City Chemicals LLC, West Haven, Connecticut USA) for 2-propylheptanol.

### Example 11:

### Preparation of Sulfuric acid mono-[2-(3,4-dihydro-isoquinolin-2-yl)-1-(iso-tridecyloxymethyl)-ethyl] ester, internal salt:

The desired product is prepared according to Example 1, substituting iso-tridecanol (obtained from BASF Corporation, Mount Olive, New Jersey USA) for 2-propylheptanol.

### Example 12:

### Simultanoeous Preparation of Sulfuric acid mono-[2-(3,4-dihydro-isoquinolin-2-yl)-1-(iso-tridecyloxymethyl)-ethyl] ester, internal salt and Sulfuric acid mono-[2-(3,4-dihydroisoquinolin-2-yl)-1-(iso-pentadecyloxymethyl)-ethyl] ester, internal salt:

The desired products are prepared according to Example 1, substituting a mixture of isomeric tridecanols to pentadecanols (obtained from BASF Corporation, Mount Olive, New Jersey USA) for 2-propylheptanol.

### Section 2:

### Example 13:

### Synthesis of:

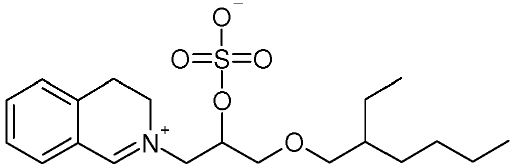

### Procedure:

3,4-dihydroisoquinoline (550 g of 94.94 % grade; 3.98 mmol dihydroisoquinoline, 1.0 eq.; 0.146 mmol isoquinoline) were dissolved in 2970 g dichloroethane. SO₃ (365.6 g, 4.54 mmol; 1.1 eq.) was added within 258 min. at a temperature of 30 to 34 °C. A yellow solid formed. The suspension was stirred for another 30 min. at 30 °C. 2-ethylhexylglycidylether (814 g, 99.15 %, 4.33 mmol, 1.05 eq., Fa. Raschig) was added within 30 min. at a temperature of 30 °C. When adding the first 30 to 40 ml a light heat tone was detectable (max T = 34 °C). The suspension was heated under reflux for 18 h, whereby a bath temperature of 90 °C was used and a temperature of 84 °C was measured within the suspension. To trap a surplus of SO₃ 65 g of dihydroisoquinoline were added after the reaction was finished.

The dichloroethane was distilled off at 850 to 700 mbar and with a temperature of the vessel of 70 to 80 °C. After stripping about 800 ml of dichloroethane a highly viscous composition came into existence. To this yellow-brown suspension ethanol (580 ml) was added and the suspension was distilled again. Distillation was performed at a pressure of 500 mbar and at a temperature of 60 to 75 °C. After stripping 1080.3 g again 580 ml of ethanol were added and the suspension was distilled again under the same conditions - this time stripping 707.3 g. To this suspension 1650 g ethanol were added and the solid, which had precipitated was dissolved at 78 °C. Then the mixture was cooled to 60 °C. At this temperature the product precipitated. The suspension was stirred (275 to 300 rpm) for 1 h at a temperature of 60 °C. Then it was cooled to 40 °C using a cooling rate of 5 °C/h and was further cooled to 0 °C using a cooling rate of 10 °C/h. The suspension was stirred over night at 0 °C - with the power of the stirrer being 0.5 W/I there was substantially no mixing within upper part of the vessel. The suspension was sucked off (time of filtration: about 500 s) and the filter cake was washed twice with cold ethanol (900 ml each). The time of filtration was 15 min. each. The filter cake having a diameter of 87 mm was dried at 50 °C and 20 mbar for three days. This yielded 1218.5 g of a light brown solid (74.3 % of theory) having a purity of 95.5 %.

### Example 14:

### Synthesis of:

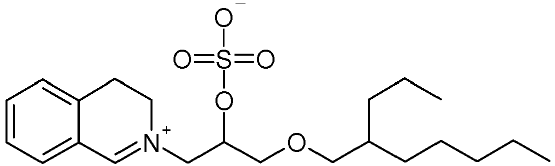

### Procedure:

### 1) 2-Propylheptyl glycidyl ether (PHGE)

In a 2 l roundbottom flask 2-propyl heptanol (316 g, 2 mol, 1.0 eq), aqueous sodium hydroxide (50 % in water, 760 g, 9.5 mol, 4.75 eq) and dimethyl cyclohexyl amin (1.7 g, 1250 ppm) were stirred (300 rpm) and heated to 50 °C. Epichloro hydrine (280 g, 237 ml, 3 mol, 1.5 eq) was added drop wise during 1 h. The resulting mixture was stirred at 50 °C for 5 h, water (714 g) was added and the phases separated (crude product contains ca. 3 % 2-propyl heptanol, ca. 78 % 2-propylheptyl glycidyl ether and higher boiling side products; gas chromatography). The organic phase was distilled (Vigreux 30 cm, 75 - 85 °C, 0.3 - 0.5 mbar):

| | |
|---|---|
| Fraction (A): | 65-75 °C, 127 g (81 % PHGE) |
| Fraction (B): | 77 °C, 172 g (96 % PHGE) |

### 2) Sulfuric acid mono-[2-(3,4-dihydro-isoquinolin-2-yl)-1-(2-propyl-heptyloxymethyl)-ethyl] ester, internal salt

3,4-dihydroisoquinoline - sulfur trioxide - complex (2.96 g, 14 mmol, 1.0 eq; prepared by addition of sulfur trioxide to dihydroisoquinoline) was dissolved under stirring at 30 °C in dioxane (14 ml). During 15 min distilled 2-propylheptyl glycidyl ether (3.2 g, 14 mmol, purity 96 %) was added at 30 °C. The suspension was heated up to 95 °C and stirred for 19 h.

The solution was treated with ethyl acetate (20 ml) and cooled down to 39 °C during 1 h, to 0 °C during the next hour, and stirred for 1 h at 0 °C afterwards. The resulting crystals were filtered off (exhausted at 90 mbar), washed with ethyl acetate (2 x 5 ml, 5 °C) and exhausted for 0.5 h at 90 mbar. After drying at 50 °C under vacuum for 12 h the desired product was obtained (3.2 g, 52 % yield).

### Example 15:

### Synthesis of:

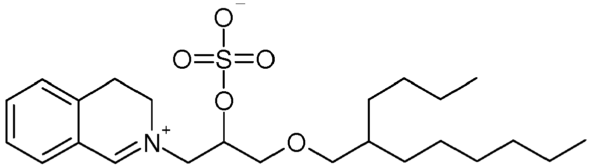

### Procedure:

Into a solution of 1707 g dichloroethane and 315 g (94.9 %ic - containing 3.8 % isoquinoline) 3,4-dihydroisoquinoline, having a temperature of 30 °C 210 g SO₃ were added within 2 h, which coursed the temperature to increase from 30 to 31 °C. After the addition the mixture was stirred for another 30 min. at a temperature of 30 °C. Within 15 min. 604 g (97.6 %ic) 2-butyloctylglycidether was added and the mixture was heated to 84 °C. After stirring for 18 h the exceeding SO₃ was trapped using 27.9 g of 3,4-dihydroisoquinoline (93.6 %ic compound containing 5.0 % isoquinoline). 897 g of dichloroethane were stripped, 333 g ethanol were added and again 359 g of solvent were stripped at 74 °C. Again 333 g ethanol were added and another 132 g of solvent were stripped at 74 °C. After another addition of ethanol (948 g) the mixture was heated to 78 °C, whereby a clear and brown solution was obtained. This solution was cooled to 70 °C within 10 min.; within the next 15 min. the temperature was decreased to 62 °C, followed by a reduction of the temperature to 51 °C within the next 15 min.; and finally a decrease in temperature to 46 °C within 8 min. lead to the beginning of crystallisation. From then on the solution was cooled to 0 °C within 1 h. After stirring for 1 h at 0 °C the crystals were sucked off and washed with cold ethanol (2 x 516 g). Drying under vacuum at 50 °C lead to light beige crystals (796.1 g; purity 97.5 %; 73.4 % of theory), which turned out to be the desired product.

### Example 16:

### Example 4 shows another synthesis route to yield mono-[2-(3,4-dihydro-isoquinolin-2-yl)-1-(2-butyl-octyloxymethyl)-ethyl] ester, internal salt:

### Procedure:

### 1) N-Formyl-N-(2-phenylethyl) amin

To a 1 I round bottom flask with 2-phenylethyl amin (500 g, 4.085 mol) methyl formiate (303 g, 4.902 mol) was added drop wise under ice-cooling during 60 min at 20-25 °C. After stirring for 30 min at 20-25 °C the reaction showed a conversion of >95 %. The slight excess of methyl formiate was removed under vacuum (1 mbar) at 60 °C giving the crude product (606 g), which was distilled (126-133 °C at ca. 0.5 mbar).

| | |
|---|---|
| Fraction (A) | 75-127 °C, 43 g (51.7 % N-formyl-N-(2-phenylethyl) amin) |
| Fraction (B) | 126-135 °C, 555 g (99.3 % N-formyl-N-(2-phenylethyl) amin) |

### 2) Dihydroisoquinoline

Polyphosphoric acid (4.42 kg) was heated to 80 °C under stirring and mixed with P₂O₅ (0.69 kg). After heating to 170 °C and stirring for 1 h at this temperature N-formyl-N-(2-phenylethyl) amin (1.32 kg, 8.84 mol) was added during 30 min. After stirring for 4 h at 170 °C the reaction was cooled down to 80 °C and carefully mixed with a 20 % solution of potassium hydroxide in water (24.75 kg) in order to result pH 7-8. The crude mixture was extracted at 60 °C with toluene (4 x 4.4 I). From the combined organic phases, which were dried over Na₂SO₄, the toluene was removed under vacuum (1 mbar) at 60 °C to give dihydroisoquinoline (1.0 kg, 85 % yield, 99.9 % purity via gas chromatography).

### 3) Sulfuric acid mono-[2-(3,4-dihydro-isoquinolin-2-yl)-1-(2-butyl-octyloxymethyl)-ethyl] ester, internal salt

In a 750 ml mini-plant vessel distilled 3,4-dihydroisoquinoline (49.85 g, 0.380 mol, 1.0 eq; purity via gas chromatography 99.9 % dihydroisoquinoline) was dissolved under stirring (400 rpm) at 30 °C in dichloro ethane (285 g). During 2 h freshly distilled sulfur trioxide (33.47 g, 17.7 ml, 0.418 mol, 1.10 eq, distilled from oleum stabilized with 0.6 weight% boric acid) was added drop wise at 30-34 °C und 30 min stirred afterwards at 30 °C. During 15 min distilled 2-butyloctyl glycidyl ether (97.04 g, 0.399 mol, purity 99.67 %) was added at 30 °C. The suspension was heated up to 84 °C and stirred for 18 h. To the brown solution 3,4-dihydroisoquinoline (5.06 g, 0.0386 mol, 0.12 eq; purity via gas chromatography 99.9 % dihydroisoquinoline) was added at 84 °C.

Dichloro ethane (160 g) was removed at 400 mbar and 55 °C, ethanol (56 g) was added, solvent mixture (129 g) was removed, ethanol (56 g) was added, solvent mixture (57 g) was removed, and ethanol (156 g) was added. After heating to 78 °C stirring was reduced (45 rpm), the solution was cooled down to 50 °C during 1 h and treated with sulfuric acid mono-[2-(3,4-dihydro-isoquinolin-2-yl)-1-(2-butyl-octyloxymethyl)-ethyl] ester internal salt (0.5 g) and stirred 1 h at 50 °C, then cooled down to 0 °C during the next hour, and stirred for 1 h at 0 °C afterwards. The resulting crystals were filtered off (exhausted at 90 mbar), washed with cold ethanol (2 x 86 g, 5 °C) and exhausted for 0.5 h at 90 mbar. After drying at 50 °C under vacuum for 12 h the desired product was obtained (135.35 g, 78.1 % yield).

### Example 17:

### Synthesis of:

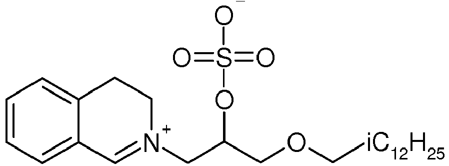

### Procedure:

### 1) Tridecyl glycidyl ether (TDGE)

In a 2 l roundbottom flask isotridekanol (500 g, 2.5 mol, 1.0 eq), aqueous sodium hydroxide (50 % in water, 950 g, 11.87 mol, 4.75 eq) and dimethyl cyclohexyl amin (2.0 g, 1250 ppm) were stirred (300 rpm) and heated to 50 °C. Epichloro hydrine (350 g, 297 ml, 3.75 mol, 1.5 eq) was added drop wise during 1 h. The resulting mixture was stirred at 50 °C for 5 h, water (1250 g) was added and the phases separated (crude product contains ca. 3 % 2-propyl heptanol, ca. 78 % 2-propylheptyl glycidyl ether and higher boiling side products; gas chromatography). The organic phase was distilled (Vigreux 30 cm, 85 - 115 °C, 0.3 - 0.5 mbar):

| | |
|---|---|
| Fraction (A): | 87-108 °C, 255 g (89 % TDGE) |
| Fraction (B): | 108-113 °C, 253 g (>99 % TDGE) |

### 2) Sulfuric acid mono-[2-(3,4-dihydro-isoquinolin-2-yl)-1-(isotridecyloxymethyl)-ethyl] ester, internal salt

3,4-dihydroisoquinoline - sulfur trioxide - complex (62.5 g, 296 mmol, 1.0 eq; prepared by addition of sulfur trioxide to dihydroisoquinoline) was dissolved under stirring at 30 °C in dioxane (261 ml). During 15 min distilled isotridecyl glycidyl ether (83.5 g, 326 mmol, purity >99 %) was added at 30 °C. The suspension was heated up to 95 °C and stirred for 17 h.

The solution was treated with ethyl acetate (390 ml) and cooled down to 39 °C during 1 h, to 0 °C during the next hour, and stirred for 1 h at 0 °C afterwards. The resulting crystals were filtered off (exhausted at 90 mbar), washed with ethyl acetate (1 x 140 ml, 5 °C) and exhausted for 0.5 h at 90 mbar. After drying at 50 °C under vacuum for 12 h the desired product was obtained (91.1 g, 62 % yield).

### Examples 18 - 37:

Also the following reaction step was varied: 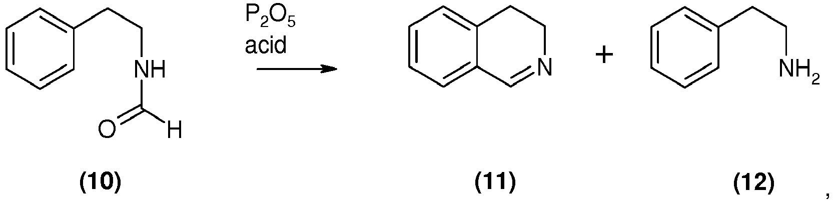 with the conditions and results being listed in table 1 below:

**Table 1:**

| starting material 30g (10) | | | | | | | | |
|---|---|---|---|---|---|---|---|---|
| | | | | | | GC [%] | | |
| **Example** | **P₂O₅** | **acid** | **solvent** | **temperature** | **time** | **10** | **11** | **12** |
| 18 | 36g | 200g PPA | none | 170°C | 40min. | | 99 | |
| 19 | 18g | 100g PPA | none | 170°C | 40min. | 7 | 91 | |
| 20 | 18g | 100g PPA | none | 170°C | 60min. | 4 | 95 | |
| 21 | 18g | 100g PPA | none | 170°C | 60min. | 3 | 92 | 4 |
| 22 | 18g | 100g PPA | none | 200°C | 60min. | | 95 | 5 |
| 23 | 18g | 100g PPA | none | 170°C | 30min. | 4 | 92 | 4 |
| 24 | 18g | 100g PPA | none | 170°C | 120min. | 1 | 94 | 4 |
| 25 | 18g | 50g PPA, 50g MSA | none | 170°C | 40min. | 31 | 36 | 1 |
| 26 | 9g | 50g PPA | none | 170°C | 40min. | 21 | 30 | 20 |
| 27 | 9g | 50g PPA | none | 200°C | 40min. | | 76 | |
| 28 | 9g | 50g PPA | dichlorobenzene | 170-180°C | 40min. | 40 | 34 | 18 |
| 29 | none | 1 eq. MSA | dichlorobenzene | 170°C | 50min. | | | 46 |
| 30 | 18g | 180g MSA | none | 130°C | 3h | 12 | 80 | 2 |
| 31 | 18g | 72g MSA | none | 130°C | 3h | 7 | 49 | 19 |
| 32 | 18g | 180g MSA | none | 115°C | 24h | 22 | 78 | |
| 33 | 18g | 180g MSA | none | 140°C | 3h | 16 | 69 | 2 |
| 34 | 18g | 180g MSA | none | 150-160°C | 2h | | 91 | |
| 35 | 18g | 90g MSA | none | 160°C | 2h | | >90 | |
| 36 | 30g | 300g MSA | none | 130°C | 4h | 6 | 81 | 1 |
| 37 | 36g | 200g formic acid | none | reflux | 4h | 100 | | |

### Examples 38 to 56:

The following reaction step was also object ob further experiments, which are summarized in table 2 below:

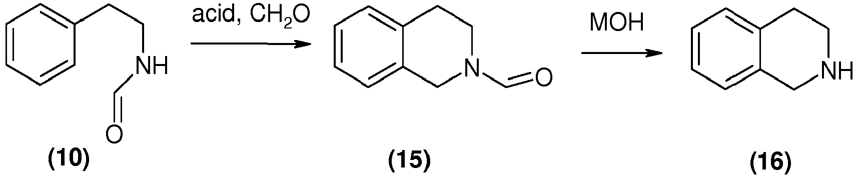

**Table 2:**

| 33 g starting material (10) | | | | | | | | |
|---|---|---|---|---|---|---|---|---|
| | | | | | | GC [%] | | |
| **Example** | **CH2O** | **solvent** | **acid** | **temperature** | **time** | **10** | **15** | **16** |
| | | | | | | | | |
| 38 | 8g paraformaldehyde | none | 220ml TFA | reflux | 4,5h | | 97 | |
| 39 | 8g paraformaldehyde | none | 100ml TFA | reflux | 4,5h | | 99 | |
| 40 | 8g paraformaldehyde | none | 75ml TFA | reflux | 4,5h | | 62 | 32 |
| 41 | 8g paraformaldehyde | none | 50ml TFA | reflux | 4,5h | | 62 | 35 |
| 42 | 8g paraformaldehyde | none | 50ml TFA | reflux | 4,5h | | 56 | 30 |
| 43 | 8g paraformaldehyde | none | 25ml TFA | reflux | 4,5h | 4 | 32 | 23 |
| 44 | 8g paraformaldehyde | none | 100ml formic acid | reflux | 4,5h | 9 | 88 | 2 |
| 45 | 8g paraformaldehyde | none | 100ml propionic acid | reflux | 4,5h | 56 | | |
| 46 | 8g paraformaldehyde | Cl(CH2)2Cl | 1eq. MSA | reflux | 4,5h | 1 | 95 | |
| 47 | 8g paraformaldehyde | Cl(CH2)2Cl | 1eq. MSA | reflux | 4,5h | | 95 | |
| 48 | 20g trioxane | none | 50ml TFA | reflux | 4,5h | | 61 | 25 |
| 49 | 20g trioxane | none | 50ml TFA | reflux | 4,5h | | 52 | 38 |
| 50 | 20g trioxane | none | 100ml formic acid | reflux | 4,5h | | 85 | 12 |
| 51 | 20g trioxane | none | 50ml TFA | reflux | 4,5h | | 63 | 34 |
| 52 | 20g trioxane | none | 50ml formic acid | reflux | 5h | 15 | 65 | 6 |
| | | | | | | | | |
| | | | | | | | | |

| | **base** | **solvent** | | | | | | |
|---|---|---|---|---|---|---|---|---|
| | | | | | | | | |
| | | | | | | | | |
| 53 | 4eq. KOH | 150ml ethanol | | reflux | 2,3h | | | 99 |
| 54 | 4eq. KOH | none | | reflux | 3,5h | | 1 | 97 |
| 55 | 1,5eq. NaOH | one | | 100°C | 5h | | 71 | 23 |
| 56 | 1,5eq. NaOH | none | | 100°C | 13,5h | | 38 | 49 |

### Section 3:

### Example 57:

The organic catalysts listed below are tested according to Applicants' Organic Catalyst/Enzyme Compatibility Test using [Peracetic Acid] = 5.0 ppm; [organic catalyst] = 0.5 ppm and the following results are obtained.

| | | Enzyme Compatibility Values | | | |
|---|---|---|---|---|---|
| Entry* | Catalyst Moiety R¹ | ECVₜₑᵣ | ECV_{dur} | ECVₙₐₜ | ECV |
| 1 | tert-butyl | 51 | 86 | 58 | 65 |
| 2 | 2-ethylhexyl | 54 | 90 | 57 | 67 |
| 3 | 2-propylheptyl | 98 | 101 | 99 | 99 |
| 4 | 2-butyloctyl | 101 | 101 | 102 | 101 |
| 5 | n-C_{12/14} | 102 | 100 | 100 | 101 |
| 6 | iso-nonyl | 86 | 96 | 88 | 90 |
| 7 | iso-decyl | 98 | 97 | 96 | 97 |
| 8 | iso-tridecyl | 99 | 100 | 101 | 100 |

| | | | | | |
|---|---|---|---|---|---|
| * Entries 1 and 2 are respectively C₄ and C₈ branched alkyl moieties, which are not encompassed by Applicants' Formula I. | | | | | |

## Claims

1. Process of producing chemical compounds of formula I 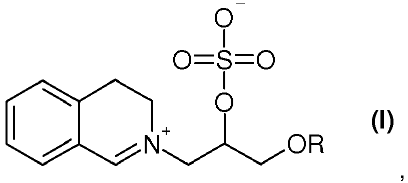 wherein R is alkyl,
comprising the following steps:
a) producing dihydroisoquinoline from isoquinoline,
b) optionally producing a glycidylether from an alkohol and an epichlorhydrine,
c) reacting said dihydroisoquinoline with SO₃ and said glycidylether,
wherein the dihydroisoquinoline in step a) is produced by
ai) reducing an isoquinoline to give a tetrahydroisoquinoline and
aii) oxidizing said tetrahydroisoquinoline to give the dihydroisoquinoline.
wherein step c) comprises steps
ci) dissolving dihydroisochinoline in a solvent,
cii) adding SO₃ to the solution obtained in ci), wherein SO₃ is used in an excess with respect to dihydroisochinoline,
ciii) adding glycidylether to the solution obtained in cii),
civ) heating the mixture obtained in ciii),
cv) quenching remaining SO₃ in the mixture obtained in ciii) or civ),
cvii) inducing crystallisation,
cviii) filtering the crystals off the mixture obtained in cvii),
cix) purifying the crystals obtained in cviii),
cx) drying the crystals obtained in cviii) or cix).

2. Process according to claim 1, wherein step a) further comprises steps
aiii) extracting the dihydroisoquinoline obtained in aii) with an organic solvent and
aiv) distilling the product obtained in aiii).

3. Process according to claim 1, wherein the glycidylether in step b) is produced by bia
) addition of an epichlorohydrine to an alcohol in the presence of a lewis acetic catalyst and subsequent reaction of the resulting chlorohydrine with NaOH and/or KOH
or
bib) reacting an epichlorohydrine with an alcohol in the presence of a phase transfer catalyst together with NaOH and/or KOH.

4. Process according to claim 3, wherein the glycidylether obtained in step bia) and/or bib) is purified by distillation.

5. Process according to claim 1, wherein step c) comprises in addition:
cvi) exchanging a substantial part of the solvent of the mixture obtained in ciii), civ) or cv).

6. Process according to claim 5, wherein:
α) the solvent in step ci) is inert with respect to SO₃ and/or
χ) step cii) takes place at a temperature of 0 °C or above and/or
δ) the glycidylether in step ciii) is used in an excess with respect to dihydroisoquinoline and/or
ε) step ciii) takes place at a temperature of 0 °C or above and/or
φ) the SO₃ in step cii) is used in greater excess with respect to the dihydroisoquinoline than is the glycidylether in step ciii) and/or
γ) heating in step civ) is performed under reflux and/or
η) the quenching in step cv) is performed using a base and/or
ηa) the amount of base used according to η) exactly matches the surplus of SO₃ or exeeds the surplus of SO₃ and/or
τ) at least 50 % of the solvent of the mixture in step cv) are exchanged and/or
ϕ) the solvent that is added in step cvi) is
an alcohol,
a mixture of alcohols or
a mixture of one or more alcohols with one or more polar aprotic solvent(s) and/or
κ) crystallization in step cvii) is induced by decreasing the temperature using a temperature ramp having zero, one or more plateaus and/or
λ) crystallization in step cvii) is induced by decreasing the temperature using a temperature ramp with the temperature being decreased at a rate of 1 to 20°C/h.

7. Process according to claim 6, wherein:
α') the solvent in step ci) is dichloroethane or dioxane or a mixture of both and/or
β') the SO₃ in step cii) is used in an amount of 1.05 to 1.15 mol per mol of dihydroisoquinoline and/or
χ') step cii) takes place at a temperature of 29 °C or above and/or
δ') the glycidylether in step ciii) is used in an amount of 1.01 to 1.1 mol per mol of dihydroisoquinoline and/or
ε') step cii) takes place at a temperature of 29 °C or above and/or
φ') the ratio of the excess of SO₃ in step cii) to the excess of the glycidylether in step ciii) - both with respect to the dihydroisoquinoline - is in the range of 1,01 - 10 : 1 and/or
γ') the temperature in step civ) is 60 °C or above and/or
η') the quenching in step cv) is performed using KOH and/or NaOH and/or aminic base and/or
η'a) the amount of KOH and/or NaOH and/or aminic base used according to η') exactly matches the surplus of SO₃ or exeeds the surplus of SO₃ and/or
τ') at least 80 % of the solvent of the mixture in step cv) are exchanged
ϕ') the alcoholic solvent that is added in step cvi) is EtOH, MeOH or iPrOH, the mixture of alcohols comprises at least one of EtOH, MeOH or iPrOH or the mixture of alcohol and polar aprotic solvent comprises acetic acid ester as the polar aprotic solvent component
and/or
κ') crystallization in step cvii) is induced by decreasing the temperature using a temperature ramp having three plateaus and/or
λ') crystallization in step cvii) is induced by decreasing the temperature using a temperature ramp with the temperature being decreased at a rate / at rates of 5 to 10 °C/h.

8. Compound according to formula I 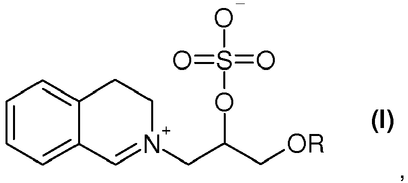 wherein R is selected from the group consisting of 2-butyloctyl, tridecanyl, 2-propylheptyl, 2-pentylnonanyl and 2-hexyldecyl.

9. Compound according to claim 8, having an enzyme compatibility value of 70 or greater.

10. Compound according to claim 9, having an enzyme compatibility value of 80 or greater.

## Patentansprüche

1. Verfahren zur Herstellung von chemischen Verbindungen der Formel I 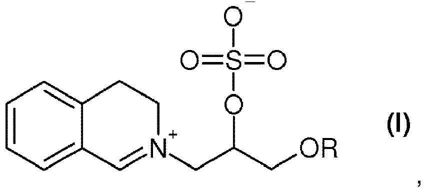 wobei R für Alkyl steht,
das die folgenden Schritte umfasst:
a) Herstellen von Dihydroisochinolin aus Isochinolin,
b) gegebenenfalls Herstellen eines Glycidylethers aus einem Alkohol und einem Epichlorhydrin,
c) Umsetzen des Dihydroisochinolins mit SO₃ und dem Glycidylether,
wobei das Dihydroisochinolin in Schritt a) durch
ai) Reduzieren eines Isochinolins zu einem Tetrahydroisochinolin und
aii) Oxidieren des Tetrahydroisochinolins zu dem Dihydroisochinolin hergestellt wird;
wobei Schritt c) folgende Schritte umfasst:
ci) Lösen von Dihydroisochinolin in einem Lösungsmittel,
cii) Zugeben von SO₃ zu der in ci) erhaltenen Lösung, wobei SO₃ im Überschuss in Bezug auf Dihydroisochinolin verwendet wird,
ciii) Zugeben von Glycidylether zu der in cii) erhaltenen Lösung,
civ) Erhitzen der in ciii) erhaltenen Mischung,
cv) Quenchen von verbliebenem SO₃ in der in ciii) oder civ) erhaltenen Mischung,
cvii) Induzieren von Kristallisation,
cviii) Abfiltrieren der Kristalle aus der in cvii) erhaltenen Mischung,
cix) Reinigen der in cviii) erhaltenen Kristalle,
cx) Trocknen der in cviii) oder cix) erhaltenen Kristalle.

2. Verfahren nach Anspruch 1, bei dem Schritt a) ferner folgende Schritte umfasst:
aiii) Extrahieren des in Schritt aii) erhaltenen Dihydroisochinolins mit einem organischen Lösungsmittel und
aiv) Destillieren des in aiii) erhaltenen Produkts.

3. Verfahren nach Anspruch 1, bei dem der Glycidylether in Schritt b) durch
bia) Zugeben eines Epichlorhydrins zu einem Alkohol in Gegenwart eines Lewis-sauren Katalysators und anschließendes Umsetzen des resultierenden Chlorhydrins mit NaOH und/oder KOH
oder
bib) Umsetzen eines Epichlorhydrins mit einem Alkohol in Gegenwart eines Phasentransferkatalysators zusammen mit NaOH und/oder KOH
hergestellt wird.

4. Verfahren nach Anspruch 3, bei dem der in Schritt bia) und/oder bib) erhaltene Glycidylether durch Destillation gereinigt wird.

5. Verfahren nach Anspruch 1, bei dem Schritt c) zusätzlich Folgendes umfasst:
cvi) Austauschen eines wesentlichen Teils des Lösungsmittels der in ciii), civ) oder cv) erhaltenen Mischung.

6. Verfahren nach Anspruch 5, bei dem:
α) das Lösungsmittel in Schritt ci) gegenüber SO₃ inert ist und/oder
χ) Schritt cii) bei einer Temperatur von 0°C oder darüber erfolgt und/oder
δ) der Glycidylether in Schritt ciii) in einem Überschuss in Bezug auf Dihydroisochinolin verwendet wird und/oder
ε) Schritt ciii) bei einer Temperatur von 0°C oder darüber erfolgt und/oder
Φ) das SO₃ in Schritt cii) in einem größeren Überschuss in Bezug auf das Dihydroisochinolin verwendet wird als der Glycidylether in Schritt ciii) und/oder
γ) das Erhitzen in Schritt civ) unter Rückfluss durchgeführt wird und/oder
η) das Quenchen in Schritt cv) mit einer Base durchgeführt wird und/oder ηa) die gemäß η) verwendete Basenmenge genau dem Überschuss an SO₃ entspricht oder über den Überschuss an SO₃ hinausgeht und/oder
τ) mindestens 50% des Lösungsmittels der Mischung in Schritt cv) ausgetauscht werden und/oder
ϕ) es sich bei dem in Schritt cvi) zugegebenen Lösungsmittel um
einen Alkohol,
eine Mischung von Alkoholen oder
eine Mischung von einem oder mehreren Alkoholen mit einem oder mehreren polaren aprotischen Lösungsmitteln handelt und/oder
κ) die Kristallisation in Schritt cvii) durch Verringerung der Temperatur unter Verwendung einer Temperaturrampe mit null, einem oder mehreren Plateaus induziert wird und/oder
λ) die Kristallisation in Schritt cvii) durch Verringerung der Temperatur unter Verwendung einer Temperaturrampe induziert wird, wobei die Temperatur mit einer Rate von 1 bis 20°C/h verringert wird.

7. Verfahren nach Anspruch 6, bei dem:
α') es sich bei dem Lösungsmittel in Schritt ci) um Dichlorethan oder Dioxan oder eine Mischung davon handelt und/oder
β') das SO₃ in Schritt cii) in einer Menge von 1,05 bis 1,15 mol pro Mol Dihydroisochinolin verwendet wird und/oder
χ') Schritt cii) bei einer Temperatur von 29°C oder darüber erfolgt und/oder
δ') der Glycidylether in Schritt ciii) in einer Menge von 1,01 bis 1,1 mol pro Mol Dihydroisochinolin verwendet wird und/oder
ε') Schritt cii) bei einer Temperatur von 29°C oder darüber erfolgt und/oder
Φ') das Verhältnis des Überschusses an SO₃ in Schritt cii) zu dem Überschuss des Glycidylethers in Schritt ciii) - jeweils in Bezug auf das Dihydroisochinolin - im Bereich von 1,01-10:1 liegt und/oder
γ') die Temperatur in Schritt civ) 60°C oder mehr beträgt und/oder
η') das Quenchen in Schritt cv) mit KOH und/oder NaOH und/oder aminischer Base durchgeführt wird und/oder
η'a) die gemäß η') verwendete Menge an KOH und/oder NaOH und/oder aminischer Base genau dem Überschuss an SO₃ entspricht oder über den Überschuss an SO₃ hinausgeht und/oder
τ') mindestens 80% des Lösungsmittels der Mischung in Schritt cv) ausgetauscht werden und/oder
Φ') es sich bei dem in Schritt cvi) zugegebenen alkoholischen Lösungsmittel um EtOH, MeOH oder iPrOH handelt,
die Mischung von Alkoholen EtOH, MeOH und/oder iPrOH umfasst oder
die Mischung von Alkohol und polarem aprotischem Lösungsmittel Essigsäureester als polare aprotische Lösungsmittelkomponente umfasst
und/oder
κ') die Kristallisation in Schritt cvii) durch Verringerung der Temperatur unter Verwendung einer Temperaturrampe mit drei Plateaus induziert wird und/oder
λ') die Kristallisation in Schritt cvii) durch Verringerung der Temperatur unter Verwendung einer Temperaturrampe induziert wird, wobei die Temperatur mit einer Rate/Raten von 5 bis 10°C/h verringert wird.

8. Verbindung der Formel I 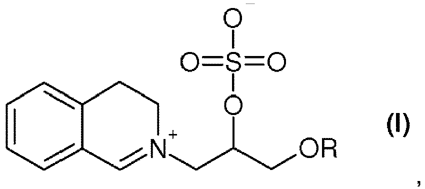 wobei R aus der Gruppe bestehend aus 2-Butyloctyl, Tridecanyl, 2-Propylheptyl, 2-Pentylnonanyl und 2-Hexyldecyl ausgewählt ist.

9. Verbindung nach Anspruch 8 mit einem Enzymverträglichkeitswert von 70 oder mehr.

10. Verbindung nach Anspruch 9 mit einem Enzymverträglichkeitswert von 80 oder mehr.

## Revendications

1. Procédé de production de composés chimiques de formule I 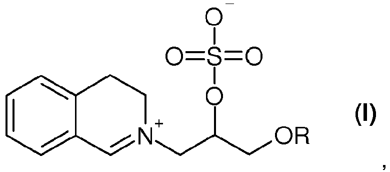 dans laquelle R représente un alkyle,
comprenant les étapes suivantes :
a) production de dihydroisoquinoléine à partir d'isoquinoléine,
b) production éventuelle d'un éther glycidylique à partir d'un alcool et d'une épichlorhydrine,
c) réaction de ladite dihydroisoquinoléine avec du SO₃ et ledit éther glycidylique,
dans lequel la dihydroisoquinoléine de l'étape a) est produite par
ai) réduction d'une isoquinoléine pour obtenir une tétrahydroisoquinoléine et par
aii) oxydation de ladite tétrahydroisoquinoléine pour obtenir la dihydroisoquinoléine,
dans lequel l'étape c) comprend les étapes suivantes :
ci) dissolution de la dihydroisoquinoléine dans un solvant,
cii) addition de SO₃ à la solution obtenue au ci), le SO₃ étant utilisé en excès par rapport à la dihydroisoquinoléine,
ciii) addition d'éther glycidylique à la solution obtenue au cii),
civ) chauffage du mélange obtenu au ciii),
cv) désactivation du SO₃ restant dans le mélange obtenu au ciii) ou au civ),
cvii) induction de la cristallisation,
cviii) prélèvement par filtration des cristaux hors du mélange obtenu au cvii),
cix) purification des cristaux obtenus au C_{V}111),
cx) séchage des cristaux obtenus au cviii) ou au cix).

2. Procédé selon la revendication 1, dans lequel l'étape a) comprend en outre les étapes suivantes :
aiii) extraction de la dihydroisoquinoléine obtenue au aii) à l'aide d'un solvant organique et
aiv) distillation du produit obtenu au aiii).

3. Procédé selon la revendication 1, dans lequel l'éther glycidylique de l'étape b) est produit par
bia) addition d'une épichlorhydrine à un alcool en présence d'un catalyseur acide de Lewis et réaction ultérieure de la chlorhydrine résultante avec du NaOH et/ou du KOH
ou par
bib) réaction d'une épichlorhydrine avec un alcool en présence d'un catalyseur de transfert de phase et de NaOH et/ou de KOH.

4. Procédé selon la revendication 3, dans lequel l'éther glycidylique obtenu à l'étape bia) et/ou bib) est purifié par distillation.

5. Procédé selon la revendication 1, dans lequel l'étape c) comprend, en plus :
cvi) l'échange d'une partie importante du solvant du mélange obtenu au ciii), civ) ou cv).

6. Procédé selon la revendication 5, dans lequel :
α) le solvant de l'étape ci) est inerte à l'égard du SO₃ et/ou
χ) l'étape cii) se déroule à une température égale ou supérieure à 0 °C et/ou
δ) l'éther glycidylique de l'étape ciii) est utilisé en excès par rapport à la dihydroisoquinoléine et/ou
ε) l'étape ciii) se déroule à une température égale ou supérieure à 0 °C et/ou
φ) le SO₃ de l'étape cii) est utilisé en plus grand excès par rapport à la dihydroisoquinoléine que l'éther glycidylique de l'étape ciii) et/ou
γ) le chauffage à l'étape civ) est mis en oeuvre à reflux et/ou
η) la désactivation à l'étape cv) est réalisée à l'aide d'une base et/ou
ηa) la quantité de base utilisée conformément au η) correspond exactement à l'excès de SO₃ ou le dépasse et/ou
τ́) au moins 50 % du solvant du mélange de l'étape cv) sont échangés et/ou
ϕ) le solvant qui est ajouté à l'étape cvi) est un alcool,
un mélange d'alcools ou
un mélange d'un ou plusieurs alcools avec un ou plusieurs solvants polaires aprotiques et/ou
κ) la cristallisation de l'étape cvii) est induite par une baisse de température utilisant une rampe de température présentant zéro, un ou plusieurs plateaux et/ou
A) la cristallisation de l'étape cvii) est induite par une baisse de température utilisant une rampe de température **caractérisée par** une baisse de température de 1 à 20 °C/h.

7. Procédé selon la revendication 6, dans lequel :
α') le solvant de l'étape ci) est le dichloroéthane ou le dioxane ou un mélange des deux et/ou
β') le SO₃ de l'étape cii) est utilisé à hauteur de 1,05 à 1,15 mole par mole de dihydroisoquinoléine et/ou
χ') l'étape cii) se déroule à une température égale ou supérieure à 29 °C et/ou
δ') l'éther glycidylique de l'étape ciii) est utilisé à hauteur de 1,01 à 1,1 mole par mole de dihydroisoquinoléine et/ou
ε') l'étape cii) se déroule à une température égale ou supérieure à 29 °C et/ou
φ') le rapport entre l'excès de SO₃ dans l'étape cii) et l'excès d'éther glycidylique dans l'étape ciii), dans les deux cas par rapport à la dihydroisoquinoléine, se situe dans la plage de 1,01-10/1 et/ou
γ') la température à l'étape civ) est égale ou supérieure à 60 °C et/ou
η') la désactivation à l'étape cv) est réalisée à l'aide de KOH et/ou de NaOH et/ou d'une base aminique et/ou
η'a) la quantité de KOH et/ou de NaOH et/ou de base aminique utilisée conformément au η') correspond exactement à l'excès de SO₃ ou le dépasse et/ou
τ́') au moins 80 % du solvant du mélange de l'étape cv) sont échangés
ϕ') le solvant alcoolique qui est ajouté à l'étape cvi) est l'EtOH, le MeOH ou l'iPrOH, le mélange d'alcools comprend au moins un alcool parmi l'EtOH, le MeOH ou l'iPrOH, ou le mélange d'alcool et de solvant polaire aprotique comprend de l'ester d'acide acétique en tant que composant de type solvant polaire aprotique
et/ou
κ') la cristallisation de l'étape cvii) est induite par une baisse de température utilisant une rampe de température présentant trois plateaux et/ou
λ') la cristallisation de l'étape cvii) est induite par une baisse de température utilisant une rampe de température **caractérisée par** une baisse/des baisses de température de 5 à 10 °C/h.

8. Composé selon la formule I 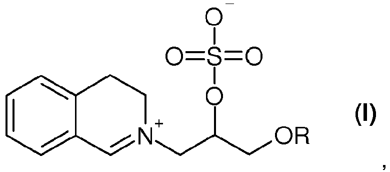 dans laquelle R est choisi dans le groupe constitué du 2-butyloctyle, du tridécanyle, du 2-propylheptyle, du 2-pentylnonanyle et du 2-hexyldécyle.

9. Composé selon la revendication 8, présentant une valeur de compatibilité enzymatique égale ou supérieure à 70.

10. Composé selon la revendication 9, présentant une valeur de compatibilité enzymatique égale ou supérieure à 80.
